# FASCICULE DE BREVET EUROPEEN

(11) **EP 0 310 522 B1**
(45) Date de publication et mention de la délivrance du brevet: **09.03.1994**
(21) Numéro de dépôt: 88402482.9
(22) Date de dépôt: 30.09.1988
(51) Int. Cl.: C12M 1/00, C12M 3/00

(54) **Dispositif de production intensive et controlée de microorganismes par photosynthèse**
Vorrichtung zur intensiven und kontrollierten Herstellung von Mikroorganismen durch Photosynthese
Device for controlled intensive production of microorganisms by photosynthesis

(30) Priorité: 02.10.1987 FR 8713647
(43) Date de publication de la demande: 05.04.1989
(73) Titulaire: COMMISSARIAT A L'ENERGIE ATOMIQUE, 75015 Paris Cédex 15 (FR)
(72) Inventeur: Berson, Xavier, F-13490 Jouques (FR); Bouyssou, Michel, F-83560 Vinon sur Verdon (FR); Castel, Yves, F-13290 Les Milles (FR); Chaumont, Daniel, F-13770 Venelles (FR); Gudin, Claude, F-13100 Aix en Provence (FR)
(74) Mandataire: Mongrédien, André

(56) Documents cités:
- FR-A- 2 324 224
- FR-A- 2 361 060
- US-A- 2 732 663

## Description

La présente invention a pour objet un dispositif de production intensive et contrôlée de micro-organismes ou biomasse, par photosynthèse, à l'échelle industrielle, comportant des composants simples et une mise en oeuvre facile.

L'invention s'applique à la production de toute matière photosynthétique, c'est-à-dire à toute forme de vie susceptible de développement et de photosynthèse dans un milieu liquide nutritif approprié en présence de rayonnement solaire et de dioxyde de carbone, à la production de métabolites et des polysaccharides éventuellement excrétés par les micro-organismes. Comme micro-organismes photosynthétiques, on peut citer les tissus végétaux et tous les organismes monocellulaires contenant des chloroplastes, les bactéries photosynthétiques et les microalgues.

La photosynthèse est la transformation, grâce à l'énergie solaire, du dioxyde de carbone en matière première hydrocarbonée, l'oxygène étant le principal sous-produit de cette transformation biochimique.

Cette réaction biochimique peut être symbolisée par l'équation de Myers suivante :

6,14 CO₂ + 3,65 H₂O + NH₃ → C_{6,14}H_{10,3}O_{2,24}N + 6,85 O₂

L'azote est apporté sous forme de nitrate, d'urée ou de sels d'ammonium introduits dans le milieu liquide contenant les micro-organismes.

La culture et le développement des micro-organismes par photosynthèse est réalisée dans un photobioréacteur. Afin d'assurer un bon fonctionnement du photobioréacteur et d'assurer un développement optimum des micro-organismes, il est nécessaire qu'un certain nombre de phénomènes physiques soit maîtrisé.

En particulier, le milieu liquide contenant les micro-organismes doit être constamment chargé en CO₂ dissous et en azote, la concentration en azote et en CO₂ dissous devant être maintenue à un niveau optimal au cours de la culture et du développement des micro-organismes. De même, la température du milieu liquide contenant les micro-organismes, sa salinité, sa concentration en cellules photosynthétiques ainsi que son pH doivent être régulés de façon rigoureuse.

Les photobioréacteurs actuellement connus sont constitués d'un ensemble de tubes transparents, disposés sous forme d'un radeau au-dessus d'une étendue d'eau importante (lagune, étang, mer, piscine) servant comme sources de refroidissement du milieu liquide chargé en micro-organismes circulant dans les tubes.

Comme photobioréacteur à structure tubulaire, on peut citer ceux décrits dans les documents FR-A-2 324 224 et FR-A-2 361 060.

Pour obtenir une bonne régulation thermique du milieu de culture, il est nécessaire d'éliminer tout type de gaz à l'intérieur des tubes du photobioréacteur, la présence statique et constante d'un gaz quel qu'il soit, et en particulier l'oxygène résultant de la photosynthèse, contrariant l'exploitation du biophotoréacteur ; d'où la nécessité d'extraire en continu les gaz et en particulier l'oxygène présents dans les tubes.

En effet, le refroidissement du milieu de culture étant assuré en immergeant tout ou partie du photobioréacteur à tubes dans l'eau sous-jacente, la présence de poches de gaz à l'intérieur de ces tubes rend difficile ou empêche leur immersion entraînant une augmentation importante de la température de ces tubes causée par le rayonnement solaire.

Ceci entraîne notamment le séchage des micro-organismes sur la paroi interne des tubes non refroidis. Le film solide de micro-organismes ainsi formé rend impossible toute diffusion de la lumière solaire, entraînant une moins bonne utilisation de l'énergie solaire et donc un mauvais rendement du photobioréacteur.

Par ailleurs, la présence d'oxygène dans les tubes peut contrarier -voire même être toxique pour- les micro-organismes cultivés dans les tubes.

Il est donc nécessaire de concevoir un dispositif de production de micro-organismes par photosynthèse empêchant toute présence de gaz et en particulier d'oxygène à l'intérieur des tubes contenant le milieu liquide chargé en micro-organismes.

Pour empêcher entre autres la formation de poches de gaz dans les tubes d'un photobioréacteur, il a été prévu l'introduction de billes en matière plastique à l'intérieur des tubes contenant le milieu de culture. Ces billes, mises en circulation avec le milieu de culture, assurent une agitation et un nettoyage des tubes par frottement sur les parois de ces derniers. Un tel système de nettoyage et de dégazage des tubes d'un photobioréacteur est notamment décrit dans le document FR-A- 2 576 034.

Si cette technique de billes autonettoyantes et dégazantes est applicable pour un photobioréacteur posé à même une surface plane rigide, son utilisation pour un photobioréacteur flottant sur une étendue d'eau est actuellement impossible.

Par ailleurs, ce système de billes auto-nettoyantes n'assure pas un dégazage efficace à 100% des tubes de culture du photobioréacteur.

L'invention a justement pour objet un dispositif de production de micro-organismes, par photosynthèse, comportant un ou plusieurs photobioréacteurs à tubes destinés à être placés sur une étendue d'eau, empêchant toute présence de poches gazeuses et notamment d'oxygène à l'intérieur des tubes.

L'invention a donc pour objet un dispositif de production intensive et contrôlée, par photosynthèse, de micro-organismes en suspension dans un milieu liquide comprenant au moins un photobioréacteur destiné à être placé sur une étendue d'eau, comportant un premier ensemble de tubes, transparents à la lumière, dans lesquels circule le milieu liquide, caractérisé en ce que le photobioréacteur comporte en outre un second ensemble de tubes disposé sous le premier ensemble, les tubes des premier et second ensembles étant rendus solidaires au moyen d'intercalaires amovibles, régulièrement espacés, ce second ensemble étant apte à rendre submersible ou insubmersible le photobioréacteur ; et en ce que le dispositif comprend aussi des moyens de commande du second ensemble de tubes en vue d'une immersion ou non du photobioréacteur ; au moins un carbonateur raccordé à l'entrée du photobioréacteur pour charger le milieu liquide en CO₂ nécessaire à la photosynthèse ; au moins un dégazeur raccordé au photobioréacteur pour éliminer du milieu liquide notamment l'oxygène produit par les micro-organismes et éventuellement le CO₂ non dissous ; et au moins une cuve d'expansion raccordée au photobioréacteur pour amortir les variations éventuelles de volume dans le photobioréacteur.

Avantageusement, les tubes du premier ensemble sont raccordés entre eux de façon à former un serpentin. De même, les tubes du second ensemble sont raccordés entre eux de façon à former un autre serpentin.

La présence des intercalaires permet de rendre parfaitement solidaires les deux ensembles de tubes du photobioréacteur et d'empêcher notamment le désalignement des tubes permettant ainsi, par entraînement linéaire du milieu liquide, jusqu'au dégazeur, la circulation des poches gazeuses éventuellement présentes dans les tubes du premier ensemble, tel que l'oxygène résultant de la photosynthèse. Dans le cas éventuel d'utilisation de billes pour nettoyer la paroi interne des tubes du premier ensemble, la présence des intercalaires favorise la circulation de ces billes.

Les intercalaires ont une mise en place facile et permettent d'éviter le collage ou le soudage des tubes entre eux, opération qui empêche le changement d'un seul tube en cas de détérioration de ce tube.

Selon l'invention, lorsque la température du liquide chargé en micro-organismes dépasse une température de consigne supérieure, le photobioréacteur est immergé grâce au dégonflement des tubes du second ensemble ; ce dégonflement est assuré à l'aide d'un automate. Inversement, lorsque la température du milieu liquide est inférieure à une température de consigne minimale, l'automate commande le gonflement des tubes du second ensemble à l'air comprimé.

L'immersion du photobioréacteur peut aussi être assurée en introduisant un liquide rélativement lourd tel que dans les tubes du second ensemble et la flottaison en injectant un fluide léger autre que de l'air.

La présence d'intercalaires, selon l'invention, améliore en outre la régulation thermique du photobioréacteur notamment lorsque les tubes du second ensemble sont destinés à être gonflés à l'air comprimé, en vue de rendre flottant le photobioréacteur, et à être dégonflés, en vue d'une immersion totale ou partielle du photobioréacteur.

En effet, en l'absence de ces intercalaires, lors de l'immersion du photobioréacteur, en vue d'un refroidissement du milieu liquide chargé en micro-organismes, les tubes du premier ensemble remplis de liquide, plus lourds que les tubes du second ensemble vides d'air, risquent de glisser entre deux tubes gonflabes et rester par conséquent en immersion constante et totale.

Afin d'améliorer le dégazage du photobioréacteur et en particulier, l'élimination de l'oxygène produit, par photosynthèse, par les micro-organismes, on utilise un dégazeur de conception tout à fait particulière.

De façon avantageuse, le dégazeur comporte une enceinte externe fermée, ayant un axe longitudinal, un conduit en U collecteur de l'oxygène dont l'une des branches est logée dans l'enceinte parallèlement audit axe et dont la base est située du côté de la base de l'enceinte, l'extrémité du conduit collectant l'oxygène débouchant dans la partie supérieure de l'enceinte, au moins une conduite d'amenée et au moins une conduite de sortie du milieu liquide débouchant dans la partie inférieure de l'enceinte.

L'utilisation d'un dégazeur selon l'invention empêche la salissure interne des tubes du photobioréacteur (par dépôt de micro-organismes) rendant ainsi inutile l'emploi de billes autonettoyantes objet du document FR-A- 2 576 034.

Comme on l'a dit précédemment, toute présence statique et constante d'un gaz quel qu'il soit à l'intérieur des tubes du premier ensemble contrarie l'exploitation du photobioréacteur. Ceci s'applique non seulement à l'oxygène produit par les micro-organismes mais aussi pour le dioxyde de carbone introduit dans le milieu liquide et qui est nécessaire au développement des micro-organismes et pour l'air éventuellement utilisé comme gaz support du gaz carbonique.

Aussi, est-il important d'utiliser un carbonateur assurant une dissolution efficace du dioxyde de carbone injecté dans le milieu liquide chargé en micro-organismes.

De préférence, le carbonateur de l'invention est un carbonateur du type à deux colonnes comportant une colonne d'entrée dans laquelle le milieu liquide se charge en CO₂ et une colonne de sortie, reliée à la colonne d'entrée, dans laquelle subverse le milieu liquide chargé en CO₂.

La première colonne a pour rôle de charger au maximum le milieu de culture en dioxyde de carbone et la seconde colonne a pour rôle d'éviter d'entraîner le dioxyde de carbone dans le photobioréacteur qui ne s'est pas dissous dans le milieu liquide et éventuellement l'air utilisé comme gaz support du CO₂.

Afin de favoriser l'enrichissement en CO₂ du milieu liquide, la colonne d'entrée comporte avantageusement un tube plongeur d'amenée du CO₂ dans le milieu liquide, dont l'extrémité plongeante est en verre ou inox fritté de porosité variable. En outre, un garnissage interne de la première colonne peut être prévu.

La ou les cuves d'expansion doivent, selon l'invention avoir une forme telle qu'il n'y ait pas accumulation de matière organique et en particulier des micro-organismes, la totalité des micro-organismes étant généralement recyclée. En outre, ces cuves doivent être transparentes à la lumière.

De façon avantageuse, la ou les cuves d'expansion sont transparentes et formées d'une enceinte à bords arrondis dont l'extrémité supérieure est fermée par un couvercle en pointe de diamant et dont l'extrémité inférieure présente la forme d'un entonnoir.

La forme et la transparence de ces cuves constituent deux moyens de lutte contre l'accumulation de biomasse où s'effectue une fermentation anaérobie et donc l'absence de zones mortes et contre la contamination par certains protistes (micro-organismes) sensibles à la lumière.

Par ailleurs, dans certains cas, la forme des cuves peut permettre d'effectuer lors de certaines contaminations une clarification de la culture : remplissage de cette cuve par de la culture, arrêt de la circulation et sédimentation. La partie surnagente contenant les contaminants (tels que des micro-algues de 5 à 10 µ) est éliminée et remplacée par du milieu de culture frais. La partie sédimentée (représentant la micro-algue cultivée) est recyclée.

D'autres caractéristiques et avantages de l'invention ressortiront mieux de la description qui va suivre, donnée à titre illustratif, en référence aux figures annexées, dans lesquelles :
- la figure 1 représente un schéma d'ensemble d'un dispositif de production de micro-organismes auquel s'applique l'invention, fonctionnant en continu.
- la figure 2 représente, de façon plus détaillée certains éléments du dispositif de la figure 1 ;
- la figure 3 représente schématiquement, en coupe longitudinale, les tubes du photobioréacteur dans lequel circule le milieu de culture, connectés entre eux, selon l'invention,
- la figure 4 représente, en perspective, les deux ensembles de tubes maintenus ensemble à l'aide d'intercalaires, selon l'invention,
- la figure 5 représente schématiquement, en coupe longitudinale, un dégazeur conforme à l'invention,
- la figure 6 représente schématiquement, en coupe longitudinale, un carbonateur conforme à l'invention,
- la figure 7 représente schématiquement, en perspective, une cuve d'expansion conforme à l'invention ; et
- la figure 8 représente schématiquement le système de régulation de l'apport en CO₂ dans les photobioréacteurs de l'invention.

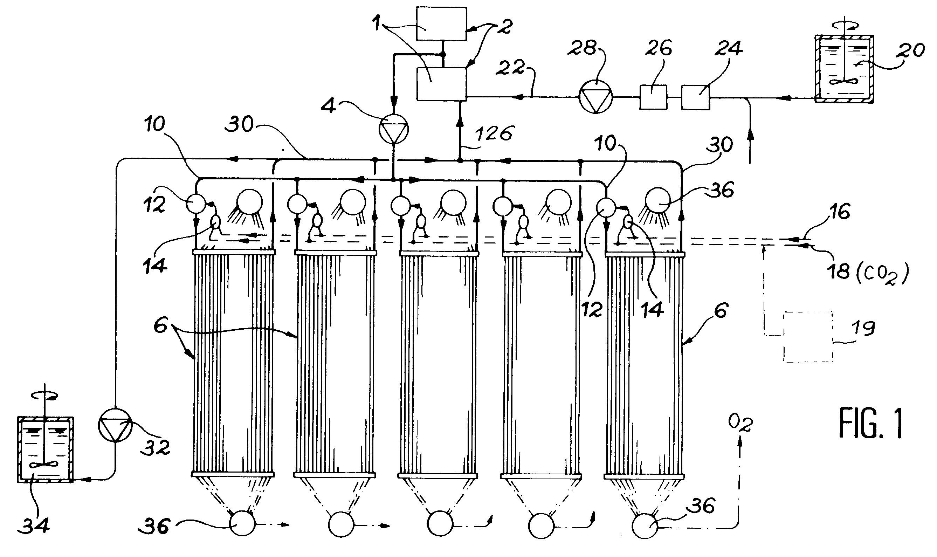

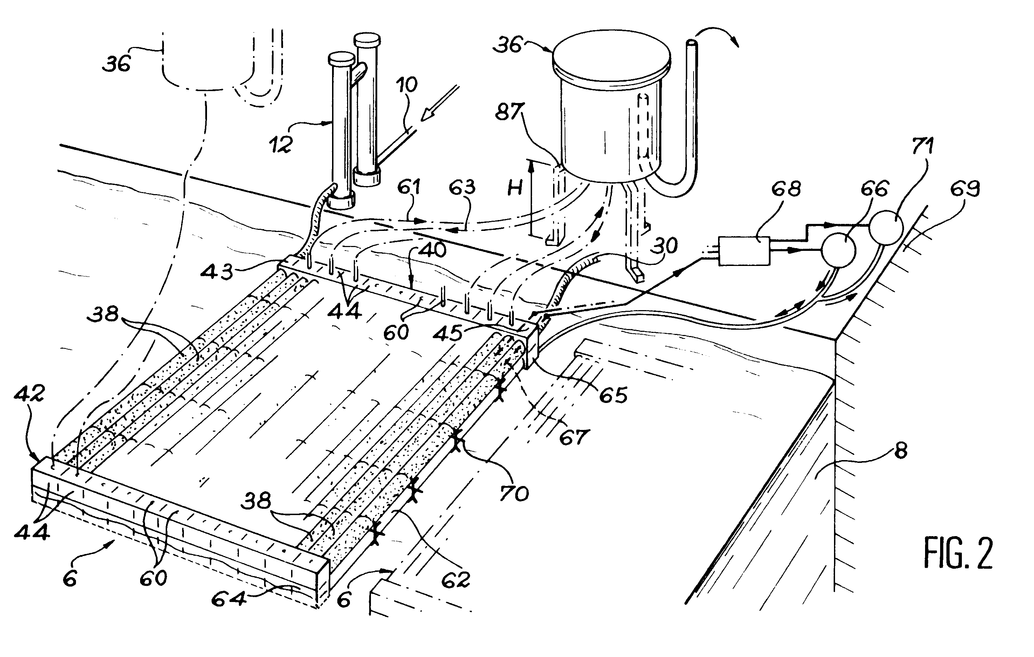

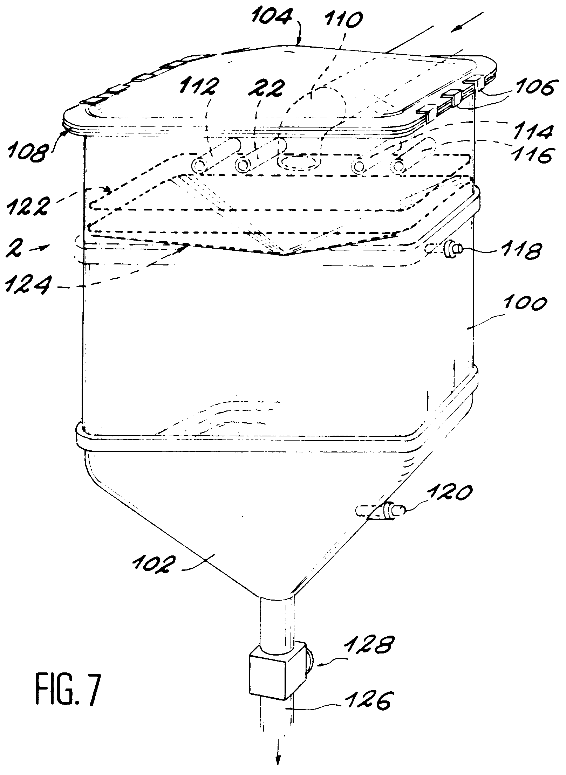

En référence aux figures 1 et 2, le dispositif de production de micro-organismes, par photosynthèse, de l'invention comprend une ou plusieurs cuves d'expansion 2 de 200 l environ destinées à recevoir une solution de microalgues 1, injectée à l'aide d'une pompe 4 dans des photobioréacteurs 6 à tubes. Ces photobioréacteurs 6 sont destinés à être placés à la surface d'une étendue d'eau 8. Le nombre de ces photobioréacteurs est fonction de la production de biomasse envisagée. Les cuves 2 servent à amortir les éventuelles variations de volume dans les photobioréacteurs dûes à l'accumulation de gaz éventuelle. La culture provenant de tous les photobioréacteurs arrivent dans ces cuves. Aussi, ces dernières constituent un volume représentatif de la totalité du milieu de culture 1 du dispositif de production. C'est à ce niveau que s'effectue l'apport en microalgues et en milieu de culture, comme on le verra ultérieurement (figure 7).

Les conduites d'amenée 10 du milieu de culture dans les photobioréacteurs sont équipées chacune de carbonateurs 12 à double colonne afin de charger le milieu de culture 1 en dioxyde de carbone nécessaire à la photosynthèse. Le nombre de carbonateurs 12 est généralement égal au nombre de photobioréacteurs.

L'apport en CO₂ est effectué en mélange avec de l'air, jusqu'à 80% en volume de CO₂ dans le mélange. A cet effet, un mélangeur-débitmètre 14, monté sur la conduite d'amenée d'air 16 et sur la conduite d'amenée 18 de CO₂ est prévu à l'entrée du gaz de chaque carbonateur 12. Un système 19 permet de réguler la quantité de CO₂ introduite dans les photobioréacteurs en fonction de l'énergie solaire.

Ce dispositif de production comprend en outre une cuve 20 servant à la préparation, sous agitation, du milieu nutritif nécessaire à la croissance des micro-organismes. Ce milieu nutritif, avant d'être introduit, via une conduite d'amenée 22 aux cuves 2 renfermant les microalgues, est filtré grâce à deux filtres 24 et 26, du type Philippe, assurant respectivement le passage des éléments minéraux et organiques, nécessaires à la croissance des micro-organismes, de 40 et 1µm. Une pompe 28 assure l'injection du milieu nutritif dans les cuves 2.

Les micro-organismes produits dans les photobioréacteurs 6 sont extraits à la sortie de chacun des photobioréacteurs, via des conduites de sortie 30, à l'aide d'une pompe volumétrique 32. Le débit de la pompe de soutirage 32 du milieu de culture 1 est égal à celui de la pompe d'injection 28 du milieu nutritif de sorte que le volume de culture dans le photobioréacteur reste constant.

Les micro-organismes produits sont récupérés dans une cuve 34, sous agitation continue.

Afin d'assurer le dégazage des tubes des photobioréacteurs et notamment l'élimination de l'oxygène produit, par photosynthèse, dans les photobioréacteurs 6, des dégazeurs 36 sont prévus de chaque côté des photobioréacteurs 6 (soit deux dégazeurs par photobioréacteur).

Selon l'invention, les photobioréacteurs sont constitués, par un premier ensemble de tubes 38, parallèles, en matière plastique souple transparente telle que du polyéthylène. Ces tubes sont destinés à recevoir le milieu de culture 1 chargé en éléments nutritifs et en CO₂ nécessaires à la croissance des micro-organismes. Ils mesurent environ 15,8 m de long et ont un diamètre interne de 63 mm.

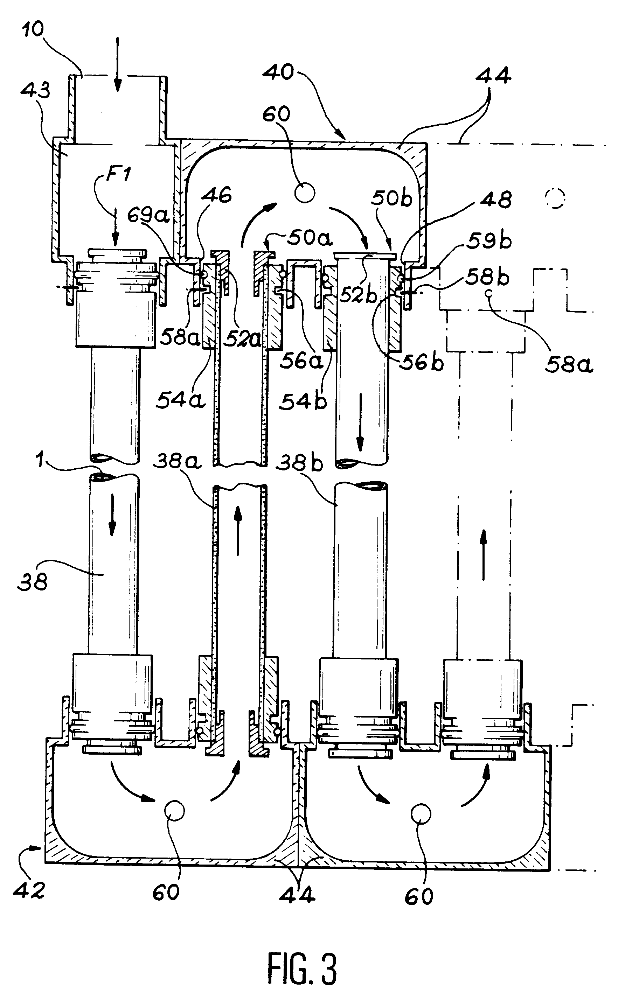

Ces tubes 38 sont connectés entre eux à leurs deux extrémités, comme représenté sur la figure 3, à l'aide de deux collecteurs respectivement amont 40 et aval 42.

Ces collecteurs 40 et 42 sont réalisés en polypropylène et comportent plusieurs compartiments ou raccords coudés 44. Le nombre total de compartiments 44 des collecteurs amont et aval est égal au nombre de tubes 38.

Chaque compartiment ou raccord 44 du collecteur amont 40 comprend un orifice d'entrée 46 destiné à recevoir un premier tube 38a et un orifice de sortie 48 destiné à recevoir un second tube 38b. La fixation des tubes 38a et 38b sur le collecteur 40 est assurée à l'aide de systèmes presse-étoupe respectivement 50a et 50b identiques.

A cet effet, deux manchons internes 52a et 52b sont logés respectivement dans les tubes 38a et 38b et deux manchons externes 54a et 54b, dans lesquels sont emmanchés les tubes 38a et 38b respectivement, sont logés respectivement dans les orifices d'entrée 46 et de sortie 48 des compartiments 44.

Les manchons externes 54a et 54b sont pourvus chacun d'une gorge 56a et 56b dans lesquelles viennent prendre place des vis 58a et 58b montées sur le raccord 44, respectivement à l'entrée et à la sortie de ce dernier.

Des joints toriques respectivement 59a et 59b, en élastomère, situés sur la face externe des manchons externes 54a et 54b, assurent l'étanchéité de l'ensemble.

Selon l'invention, les manchons internes 52a, 52b et les manchons externes 54a, 54b sont réalisés en polypropylène.

L'assemblage du collecteur aval 42 et des tubes 38 est identique en tout point à celui du collecteur amont 40 et des tubes 38.

A l'intérieur du serpentin ainsi formé circule le milieu de culture 1 chargé en CO₂ et en éléments nutritifs, comme l'indiquent les flèches F1.

Chaque photobioréacteur 6 comporte une unique conduite d'amenée 10 du milieu de culture, montée à l'unique entrée 43 du collecteur amont 40, et une unique conduite de sortie 30 dudit milieu, montée à l'unique sortie 45 du collecteur 40, la conduite d'entrée 10 étant montée sur le premier tube 38 du photobioréacteur et la conduite de sortie 30 sur le dernier tube 38 de ce même photobioréacteur.

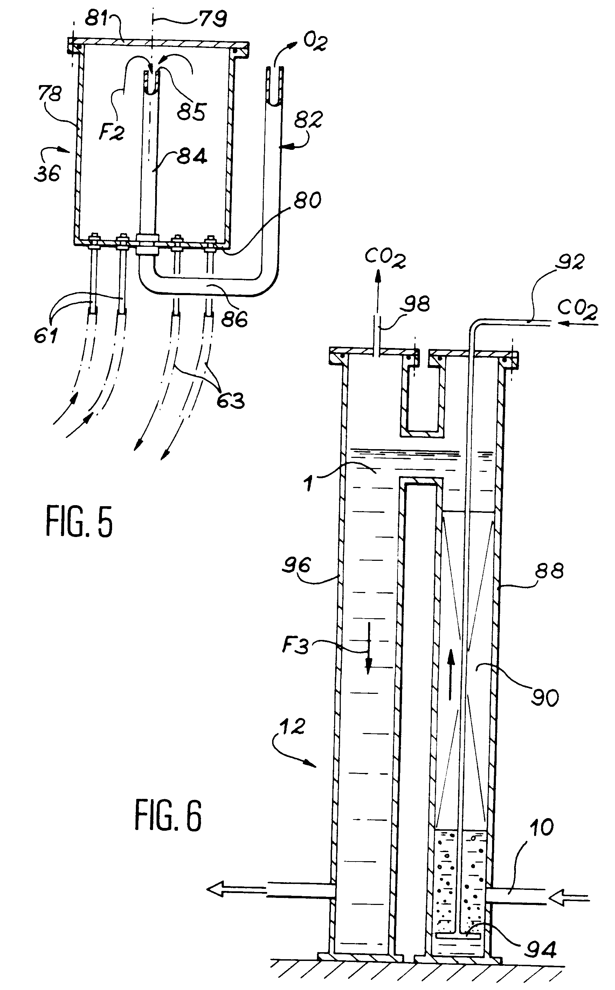

Selon l'invention, chaque raccord 44 du collecteur amont 40 et aval 42 est pourvu d'un orifice 60 servant au branchement des dégazeurs 36. Ce branchement est assuré à l'aide de conduites souples d'entrée 61 et de sortie 63 du milieu de culture (figures 2 et 5).

La solution de micro-organismes a besoin pour sa propre croissance d'être régulée à une température allant de 18 à 28°C, c'est-à-dire proche de la température ambiante.

A cet effet, chaque photobioréacteur 6 est pourvu d'un second ensemble de tubes 62 placé en-dessous des tubes 38. Ces tubes 62 en matière plastique souple, par exemple en polyéthylène, sont connectés entre eux comme les tubes 38, à l'aide d'un collecteur amont 64 et d'un collecteur aval 65, à compartiments coudés, pour constituer un serpentin (figure 2). Ces tubes 62, de 16,4 m de long et 63 mm de diamètre interne sont destinés à être gonflés à l'air comprimé, à l'aide d'une électrovanne 66, formant ainsi un support pneumatique flottant à la surface de l'étendue d'eau 8.

La commande de l'électrovanne 66 est assurée par un circuit électronique de commande 68 relié à des sondes 67 de mesure thermique logées à l'intérieur des tubes 38.

Lorsque la température du milieu de culture 1 contenue dans les tubes 38, mesurée par les sondes 67, est supérieure à une température de consigne maximale (29°C par exemple), les sondes thermiques émettent un premier signal détecté par le circuit électronique 68 commandant alors le dégonflage des tubes 62 par l'intermédiaire des électrovannes 71. Le dégonflage des tubes 62 entraîne alors l'immersion totale ou partielle, suivant le taux de dégonflage, des photobioréacteurs 6 dans l'étendue d'eau 8. L'eau étant plus froide que l'atmosphère ambiante et que le milieu de culture présent dans les photobioréacteurs, le milieu de culture se refroidit.

Lorsque le milieu de culture immergé atteint une température, mesurée par les sondes 67, inférieure à une température de consigne minimale (par exemple 17°C), les sondes 67 émettent un second signal détecté par le circuit électronique 68 commandant alors l'électrovanne 66 afin d'assurer le gonflement en air comprimé des tubes 62. Le gonflement des tubes 62 entraîne alors la flottaison des photobioréacteurs 6. Le milieu de culture se réchauffe alors à l'air libre.

Toutes les conduites d'amenée et de sortie du milieu de culture, d'air comprimé etc... et toutes les connexions électriques (sondes 67), branchées entre les photobioréacteurs et les autres composants du dispositif de production tels que les dégazeurs, les carbonateurs, les compresseurs etc... sont nécessairement souples pour être compatibles avec l'immersion ou non des photobioréacteurs. Les composants autres que les photobioréacteurs sont montés sur un ponton en dur 69 comme schématisé sur la figure 2, flottant à la surface de l'eau 8.

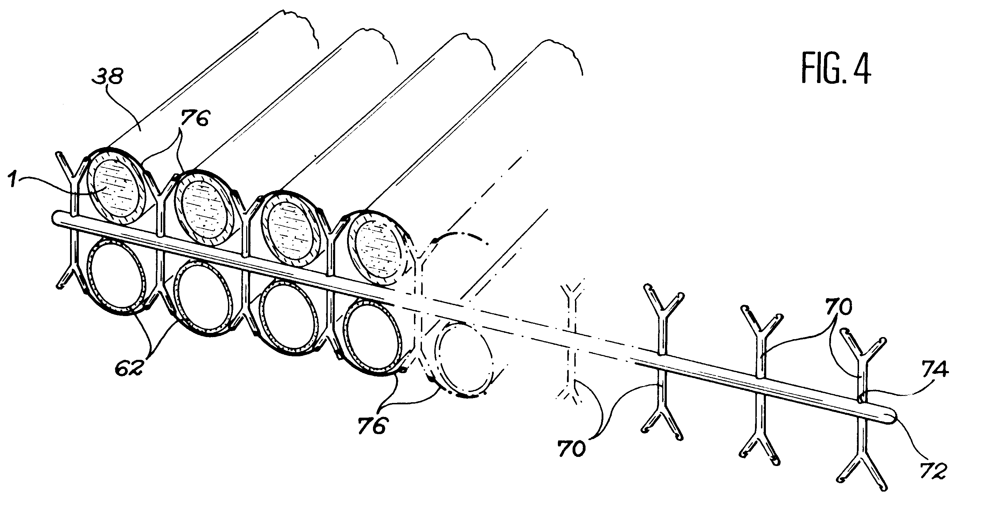

Conformément à l'invention, des intercalaires 70, représentés sur les figures 2 et 4, assurent le maintien en place des tubes 38 et 62. Ces intercalaires 70 sont amovibles et régulièrement espacés, tous les 60 cm environ (figure 2). Ces intercalaires 70 sont réalisés en polypropylène et présentent la forme de Y dont les queues sont supportées par des tiges supports 72 notamment en polypropylène. A cet effet, les supports 72 sont équipés d'orifices 74 dans lesquels sont logées les queues des Y 70. Les Y disposés de part et d'autre des tiges 72 et servant respectivement au maintien des tubes 38 et 62 sont fixés tête-bêche.

Les Y 70 sont reliés entre eux à l'aide de liaisons souples 76 en caoutchouc, montées sur les branches des Y. Les tubes 38 et 62 sont disposés entre deux Y 70 consécutifs, leur maintien en place étant assuré par les liaisons élastiques 76 et le support 72.

La régulation thermique décrite précédemment ainsi que le bon fonctionnement des photobioréacteurs 6 impliquent l'absence totale de gaz à l'intérieur des tubes 38 des photobioréacteurs. A cet effet, on utilise des dégazeurs 36, réalisés avantageusement comme représentés sur la figure 5 et branchés à chaque extrémité des tubes 38.

Chaque dégazeur 36 comporte une enceinte externe 78 de forme cylindrique, comportant un axe longitudinal 79, pourvue d'un fond 80 et fermée par un couvercle 81, ainsi qu'un conduit en U 82 servant à collecter l'oxygène et les autres gaz présents dans le milieu de culture 1. Ce tube en U 82 comporte une branche 84 logée selon l'axe longitudinal 79 de l'enceinte 78. L'extrémité 85 de cette branche 84 collectant l'oxygène provenant du milieu de culture débouche dans la partie supérieure de l'enceinte 78, à proximité du couvercle 81. La base 86 du conduit en U est parallèle au fond 80 de l'enceinte 78. Le sens de circulation de l'oxygène dans le dégazeur 36 est indiqué par les flèches F2.

Les conduites souples d'amenée 61 du milieu de culture chargé en oxygène (généralement 60% en volume d'oxygène) et les conduites souples de sortie 63 du milieu de culture désoxygéné et plus généralement dégazé débouche à la base du dégazeur et sont fixées au fond 80 de l'enceinte 78.

Les dégazeurs 36, comme indiqué sur la figure 2 sont supportés par des pieds 87 dont la hauteur H est réglée afin que l'enceinte 78 des dégazeurs ne soit jamais remplie par du milieu de culture, que les photobioréacteurs soient immergés ou flottent. La hauteur de culture dans les tubes 63 est déterminée par la pression interne dans les tubes 38.

Les micro-organismes du milieu de culture 1 ont un besoin permanent en CO₂ d'où la nécessité de charger constamment le milieu de culture en CO₂ dissous. Afin d'assurer le maximum de charge en CO₂ du milieu de culture, on utilise, conformément à l'invention, un carbonateur 12 connecté à l'entrée de chaque photobioréacteur tel que représenté sur la figure 6.

Ce carbonateur comporte une colonne d'entrée 88 cylindrique, fermée à ses deux extrémités et contenant éventuellement un garnissage 90 du type anneaux de Raschig ou selles de Bert. Dans cette colonne 88, le milieu de culture, arrivant à la base de la colonne 88, se charge en CO₂ introduit à l'aide d'un tube plongeur 92 dont l'extrémité plongeante 94 est réalisée en verre fritté de porosité variable afin d'améliorer la dissolution du CO₂ dans le milieu liquide 1.

Cette colonne d'entrée 88 est reliée à sa partie supérieure à une colonne de sortie 96 dans laquelle subverse le milieu liquide chargé en CO₂. Cette colonne 96 a pour but d'éviter l'entraînement du CO₂ et éventuellement du gaz support non dissous dans le milieu de culture vers le photobioréacteur associé. Une conduite 98 d'évacuation du CO₂ non dissous est prévue au sommet de la colonne de sortie 96. Les flèches F3 indiquent le sens de circulation du milieu liquide 1 dans le carbonateur.

Les cuves d'expansion 2, comme les autres parties du dispositif de production, contribuent aussi à empêcher le dépôt de micro-organismes dans les tubes 38 des photobioréacteurs et donc à améliorer le rendement des photobioréacteurs. A cet effet, on utilise avantageusement des cuves telles que représentées sur la figure 7.

La cuve représentée comporte une enceinte 100 parallélépipédique dont l'extrémité inférieure 102 présente la forme d'un entonnoir ; cette cuve a la forme d'une trémie ou d'un silo et son fond évasé permet la sédimentation et le recyclage total de la matière organique en suspension (micro-organismes + produits excrétés par les micro-organismes).

La cuve ne comporte aucun bord ou arrête vive et les angles sont arrondis afin d'éviter une fixation excessive des micro-organismes et faciliter son nettoyage. Elle comporte en outre un couvercle 104 en pointe de diamant afin d'éviter l'accumulation de condensation ; la pointe du couvercle est dirigée vers le haut de la cuve.

Le couvercle 104 est fixé à l'enceinte 100 par des clips métalliques 106 ou des vis papillon. Un joint torique 108 assure l'étanchéité entre l'enceinte et le couvercle.

L'enceinte 100 de la cuve et son couvercle 104 sont transparents afin de permettre l'observation mais aussi de lutter contre les contaminations de certains protistes sensibles à la lumière ; elle est réalisée en Plexiglass^{R} ou en PVC (polychlorure de vinyle).

Dans sa partie supérieure, une arrivée 110 de la culture (micro-organismes) permet un remplissage central de la trémie en micro-organismes. En outre, des conduites d'amenée 112, 22, 114 et 116 sont prévues pour introduire respectivement dans la cuve 2 le milieu de culture lors du remplissage du dispositif, le milieu de culture lors du fonctionnement en continu, le régulateur de pH (acide ou basique) et l'agent antimousse.

Une sonde 118 de niveau haut et une sonde 120 de niveau bas permettent d'empêcher respectivement le débordement ou le vidage complet de la cuve 2 pendant le fonctionnement en continu du dispositif général de production.

A la partie supérieure de la cuve une première grille métallique plane 122 et une seconde grille métallique 124 sont prévues. La première grille, de 1 cm de maille permet, lors de l'utilisation éventuelle de billes de polyuréthane d'autonettoyage, la récupération de ces billes. La seconde grille, de 1 mm de maille, en pointe de diamant, permet la récupération des gros agrégats cellulaires provenant de l'autonettoyage par les billes ; la pointe de la grille est dirigée vers le fond de la cuve.

Le départ du milieu de culture vers les photobioréacteurs est assuré par la conduite 126, à l'extrémité inférieure 102 de la cuve, une vanne d'arrêt 128 étant montée sur cette conduite 126.

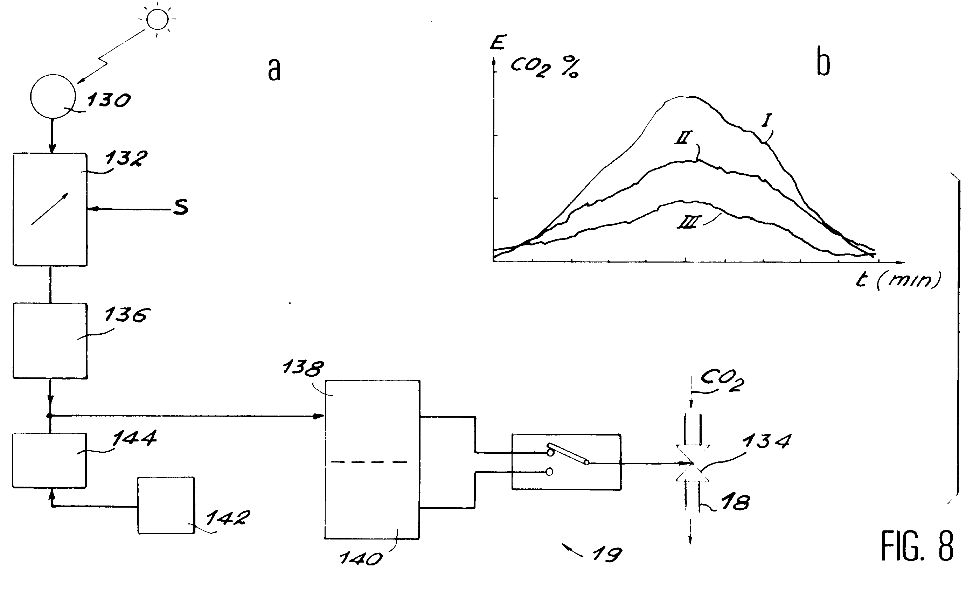

Comme on l'a dit précédemment, une régulation 19 de l'alimentation en CO₂ du milieu de culture en fonction de l'ensoleillement est prévue. Ce système de régulation 19 est représenté sur la figure 8, partie a.

Il comporte un pyranomètre 130 délivrant une tension électrique de 0 à 10 mV représentant les variations de l'énergie lumineuse. Ce signal électrique est amplifié à l'aide d'un amplificateur à gain variable 132, entre 0 et 5V. L'amplification maximum représente le débit de la vanne 134 à pleine ouverture, montée sur la conduite 18 (figure 1). Un circuit 136 du type RC permet d'amortir la réponse de la vanne 134 aux variations lumineuses de très courte durée.

L'utilisation d'un amplificateur 132 à gain variable permet une modification de la sensibilité du système 19, c'est-à-dire le choix du niveau d'intensité lumineuse déterminant l'ouverture complète de la vanne 134. Un réglage du seuil S de zéro permet de choisir le seuil d'énergie solaire déclenchant l'ouverture de la vanne 134 ; cette adaptation permet de tenir compte du point de compensation des micro-organismes photosynthétiques.

Le signal amplifié sortant du circuit 136 est dirigé vers un circuit électronique 138 de commande de vanne, la vanne 134 étant une vanne électronique modèle 5850TR. La vanne peut être commandée manuellement par une alimentation extérieure 140 (0-5V) ou bien automatiquement grâce à un ordinateur 142. Un circuit adaptateur 144 est prévu entre l'ordinateur et le circuit RC.

Ce système de régulation 19 permet, comme représenté sur la partie b de la figure 8, d'alimenter le milieu de culture en CO₂ en fonction des variations de l'énergie lumineuse E au cours du temps t, exprimé en minute. La courbe I représente les variations de l'énergie solaire, la courbe II représente les variations de l'alimentation en CO₂ du milieu de culture et la courbe III représente les variations du CO₂ non consommé.

L'activité photosynthétique (fixation du CO₂ par les micro-organismes) est proportionnelle à l'intensité lumineuse. Or l'intensité du flux solaire est variable au cours de la journée : minimale le matin et le soir, maximale lorsque le soleil est au zénith. Une alimentation constante en CO₂ aboutit donc le matin et le soir à des pertes importantes en CO₂ (faible activité photosynthétique) et parfois à une limitation de la photosynthèse par manque de CO₂ lorsque l'intensité lumineuse est maximale.

La régulation en CO₂ permet donc une économie en CO₂ puisque sa fourniture est proportionnelle à la demande biologique.

Le dispositif de production selon l'invention peut être utilisé pour le développement et la culture de cellules de plantes ou d'algues unicellulaires telles que celles décrites dans la publication de G. Gudin et C. Thépenier "Bioconversion of solar energy into organic chemicals by microalgae" paru dans Advances in Biotechnological Processes 6, pp. 73-110 de 1986.

Les milieux nutritifs de ces dernières contiennent divers sels minéraux, sels métalliques, acides aminés, vitamines et régulateurs de croissance. Des exemples d'éléments nutritifs utilisables dans l'invention sont notamment illustrés dans les documents FR-A-2 324 224 et FR-A-2 361 060.

Le dispositif de production décrit précédemment permet notamment une production industrielle de 60 tonnes par an environ de biomasse telle que Porphyridium cruentum, Scenedesmus acutus, Dunalulla, et ce, de façon industrielle, en utilisant des photobioréacteurs tubulaires répartis sur une surface d'eau de 1 ha, avec une exploitation de 300 jours par an, une période d'éclairement solaire de 12 h en moyenne par jour (ce qui correspond à une énergie solaire de 950 W/m²), une quantité de CO₂ fixe de 56 000 m³ par an, soit 110 tonnes par an, et une quantité de milieu nutritif par jour de 50 m³, ce qui correspond à 30kg d'urée par jour environ, la quantité d'oxygène rejetée dans l'atmosphère étant de 56 000 m³ par an soit 154 tonnes par an.

## Revendications

1. Dispositif de production intensive et contrôlée, par photosynthèse, de micro-organismes en suspension dans un milieu liquide comprenant au moins un photobioréacteur (6) destiné à être placé sur une étendue d'eau (8), comportant un premier ensemble de tubes (38), transparents à la lumière, dans lesquels circule le milieu liquide (1), caractérisé en ce que le photobioréacteur comporte en outre un second ensemble de tubes (62) disposé sous le premier ensemble, les tubes des premier et second ensembles étant rendus solidaires au moyen d'intercalaires (70) amovibles, régulièrement espacés, ce second ensemble étant apte à rendre submersible ou inssubmersible le photobioréacteur ; et en ce que le dispositif comprend aussi des moyens de commande (66, 67, 68) du second ensemble de tubes en vue d'une immersion ou non du photobioréacteur (6) ; au moins un carbonateur (12) raccordé à l'entrée (10) du photobioréacteur (6) pour charger le milieu liquide en CO₂ nécessaire à la photosynthèse ; au moins un dégazeur (36) raccordé au photobioréacteur pour éliminer du milieu liquide (1) notamment l'oxygène produit par les micro-organismes ; et au moins une cuve d'expansion (2) raccordée au photobioréacteur (6) pour amortir les variations éventuelles de volume dans le photobioréacteur (6).

2. Dispositif selon la revendication 1, caractérisé en ce que les intercalaires (70) présentent la forme d'Y montés par leur queue sur au moins un support rigide (72) disposé entre les premier et second ensembles de tubes et sont reliés entre eux à l'aide de liaisons souples (76), par leurs branches, les tubes (38, 62) des premier et second ensembles étant disposés entre deux Y (70).

3. Dispositif selon la revendication 1 ou 2, caractérisé en ce que les tubes (38) du premier ensemble sont raccordés entre eux de façon à former un serpentin, les extrémités des tubes étant pourvues de raccords (44) auxquels est raccordé le dégazeur (36).

4. Dispositif selon l'une quelconque des revendications 1 à 3, caractérisé en ce que les tubes (62) du second ensemble sont raccordés entre eux de façon à former un serpentin.

5. Dispositif selon la revendication 3, caractérisé en ce que les raccords (44) sont fixés aux tubes du premier ensemble chacun à l'aide d'un premier (52a) et d'un second (52b) manchons internes, montés dans respectivement un premier (38a) et un second (38b) tubes du premier ensemble ; d'un premier (54a) et d'un second (54b) manchons externes dans chacun desquels sont logés respectivement lesdits premier et second tubes, les premier et second manchons internes étant rendus solidaires respectivement des premier et second manchons externes.

6. Dispositif selon la revendication 5, caractérisé en ce que les tubes (38) du premier ensemble sont réalisés en polyéthylène et les manchons internes (52a, 52b) et externes (54a, 54b) en polypropylène.

7. Dispositif selon l'une quelconque des revendications 1 à 6, caractérisé en ce que les intercalaires (70) sont réalisés en polypropylène.

8. Dispositif selon l'une quelconque des revendications 1 à 7, caractérisé en ce que le carbonateur est un carbonateur (12) du type à deux colonnes comportant une colonne d'entrée (88) dans laquelle le milieu liquide (1) se charge en CO₂ et une colonne de sortie (96), reliée à la colonne d'entrée, dans laquelle subverse le milieu liquide chargé en CO₂.

9. Dispositif selon la revendication 8, caractérisé en ce que la colonne d'entrée (88) comporte un tube plongeur (92) d'amenée du CO₂ dans le milieu liquide (1), dont l'extrémité plongeante (94) est en verre ou inox fritté de porosité variable.

10. Dispositif selon la revendication 8 ou 9, caractérisé en ce que la colonne d'entrée (88) est pourvue d'un garnissage (90) pour favoriser l'introduction du CO₂ dans le milieu liquide (1).

11. Dispositif selon l'une quelconque des revendications 1 à 10, caractérisé en ce que le dégazeur (36) comporte une enceinte externe fermée (78), ayant un axe longitudinal (79), un conduit en U (82) collecteur de l'oxygène dont l'une des branches (84) est logée dans l'enceinte (78) parallèlement audit axe et dont la base (86) est logée du côté de la base (80) de l'enceinte, l'extrémité (85) du conduit (82) collectant l'oxygène débouchant dans la partie supérieure de l'enceinte ; au moins une conduite d'amenée (61) et au moins une conduite de sortie (63) du milieu liquide (1) débouchant dans la partie inférieure de l'enceinte.

12. Dispositif selon l'une quelconque des revendications 1 à 11, caractérisé en ce que les moyens de commande (66, 67, 68) du second ensemble de tubes (38) sont du type pneumatique et servent à gonfler et dégonfler les tubes (62) du second ensemble respectivement lorsque la température du milieu liquide (1) est inférieure à une première température de consigne et supérieure à une seconde température de consigne, la première température de consigne étant inférieure à la seconde température de consigne.

13. Dispositif selon l'une quelconque des revendications 1 à 12, caractérisé en ce que la cuve d'expansion (2) est transparente et formée d'une enceinte (100) à bords arrondis dont l'extrémité supérieure est fermée par un couvercle (104) en pointe de diamant et dont l'extrémité inférieure présente la forme d'un entonnoir (102).

14. Dispositif selon la revendication 13, caractérisé en ce que la cuve est équipée dans sa partie supérieure d'une grille métallique (124) en pointe de diamant.

15. Dispositif selon l'une quelconque des revendications 1 à 14, caractérisé en ce qu'il comprend des moyens (19) de régulation de la quantité de CO₂ introduit dans le photobioréacteur (6) en fonction de l'énergie solaire.

## Claims

1. Apparatus for the intensive, controlled production by photosynthesis of microorganisms suspended in a liquid medium comprising at least one photobioreactor (6) which is to be placed on an expanse of water (8) , having a first group of tubes (38), which are transparent to the light and in which circulates the liquid medium (1), characterized in that the photobioreactor also has a second group of tubes (62) placed beneath the first group, the tubes of the first and second groups being joined together by detachable interpolated members (70) which are regularly spaced, said second group being able to make the photobioreactor submersible or non-submersible, in that the apparatus also comprises means (66, 67, 68) for controlling the second group of tubes with a view to the immersion or not of the photobioreactor (6), at least one carbonator (12) connected to the intake (10) of the photobioreactor (6) for charging the liquid medium with the CO₂ necessary for the photosynthesis, at least one degassing means (36) connected to the photobioreactor in order to eliminate from the liquid medium (1) the oxygen produced by the microorganisms and optionally the undissolved CO₂ and at least one expansion tank (2) connected to the photobioreactor (6) in order to reduce possible variations of the volume of the photobioreactor (6).

2. Apparatus according to claim 1, characterized in that the interpolated members (70) are Y-shaped and that the tail thereof is mounted on at least one rigid support (72) placed between the first and second groups of tubes and which are interconnected with the aid of flexible connections (76), by their branches, the tubes (38, 62) of the first and second groups being placed between two Y's (70).

3. Apparatus according to claims 1 or 2, characterized in that the tubes (38) of the first group are interconnected so as to form a coil, the ends of the tubes being provided with connections (44) to which is connected the degassing means (36).

4. Apparatus according to any one of the claims 1 to 3, characterized in that the tube (62) of the second group are interconnected so as to form a coil.

5. Apparatus according to claim 3, characterized in that the connections (44) are fixed to the tubes of the first group, each with the aid of a first (52a) and a second (52b) inner sleeves, mounted in respectively a first (38a) and a second (38b) tube of the first group, a first (54a) and a second (54b) outer sleeves, in each of which are respectively located said first and second tubes, the first and second inner tubes being rendered integral respectively with the first and second outer sleeves.

6. Apparatus according to claim 5, characterized in that the tubes (38) of the first group are made from polyethylene and the inner sleeves (52a, 52b) and the outer sleeves (54a, 54b) are made from polypropylene.

7. Apparatus according to any one of the claims 1 to 6, characterized in that the interpolated members (70) are made from polypropylene.

8. Apparatus according to any one of the claims 1 to 7, characterized in that the carbonator is a carbonator (12) having two columns with an intake column (88) where the liquid medium (1) is charged with CO₂ and a discharge column (96), connected to the intake column into which is introduced the CO₂-charged liquid medium.

9. Apparatus according to claim 8, characterized in that the intake column (88) has a plunger tube (92) for supplying CO₂ to the liquid medium (1), whose plunging end (94) is of variable porosity fritted stainless steel or glass.

10. Apparatus according to claims 8 or 9, characterized in that the intake column (88) is provided with a lining (90) for assisting the introduction of CO₂ into the liquid medium (1).

11. Apparatus according to any one of the claims 1 to 10, characterized in that the degassing means (36) has a sealed outer enclosure (78), which has a longitudinal axis (79), an oxygen collecting Y-pipe (82), whereof one of the branches (84) is located in the enclosure (78) parallel to said axis and whose base (86) is located on the side of the base (80) of the enclosure, the end (85) of the oxygen-collecting pipe (82) issuing into the upper part of the enclosure and at least one supply pipe (61) and at least one discharge pipe (63) for the liquid medium (1) issuing into the lower part of the enclosure.

12. Apparatus according to any one of the claims 1 to 11, characterized in that the control means (66, 67, 68) of the second group of tubes (38) are of the pneumatic type and are used for inflating and deflating the tube (62) of the second group respectively when the temperature of the liquid medium (1) is below a first reference temperature and above a second reference temperature, the first reference temperature being below the second reference temperature.

13. Apparatus according to any one of the claims 1 to 12, characterized in that the expansion tank (2) is transparent and formed from an enclosure (100) having rounded edges, whose upper end is closed by a diamond tip cover (104) and whose lower end is shaped like a funnel (102).

14. Apparatus according to claim 13, characterized in that the upper part of the tank is provided with a diamond tip metal grid (124).

15. Apparatus according to any one of the claims 1 to 14, characterized in that it comprises means (19) for regulating the quantity of CO₂ introduced into the photobioreactor (6) as a function of the solar energy.

## Patentansprüche

1. Vorrichtung zur intensiven und kontrollierten Herstellung, durch Photosynthese, von Mikroorganismen in Suspension in einem flüssigen Milieu, welche mindestens einen Photobioreaktor (6) umfaßt, der dazu bestimmt ist, auf einer Wasserfläche (8) angeordnet zu sein, der eine erste Röhreneinheit (38) umfaßt, die bei Licht transparent sind und in welchen das flüssige Milieu (1) zirkuliert, dadurch gekennzeichnet, daß der Photobioreaktor außerdem eine zweite Röhreneinheit (62) umfaßt, die unter der ersten Einheit gelagert ist, wobei die Röhren der ersten und der zweiten Einheit mithilfe von abnehmbaren Zwischenlagen (70) mit regelmäßigen Abständen festgehalten werden, diese zweite Einheit ist fähig, den Photobioreaktor in Wasser versenkbar oder nicht versenkbar zu machen; und daß die Vorrichtung auch Geräte zur Steuerung (66, 67, 68) der zweiten Röhreneinheit hinsichtlich dem Eintauchen oder nicht Eintauchen des Photobioreaktors (6) umfaßt; mindestens einen Karbonateur (12) der am Eingang (10) des Photobioreaktors (6) angeschlossen ist, um das flüssige Milieu mit dem für die Photosynthese notwendigen CO₂ zu beladen; mindestens einem Entgaser (36), der an dem Photobioreaktor angeschlossen ist, um aus dem flüssigen Milieu (1) besonders den von den Mikroorganismen produzierten Sauerstoff zu beseitigen; und mindestens einen Ausdehnungsbehälter (2), der an dem Photobioreaktor (6) angeschlossen ist, um die eventuellen Volumenänderungen in dem Photobioreaktor (6) auszugleichen.

2. Verfahren gemäß Anspruch 1, dadurch gekennzeichnet, daß die Zwischenlagen (70), welche eine Y-Form aufweisen, mit ihrem Endstück auf mindestens einer starre Stütze (72) montiert sind, die zwischen der ersten und der zweiten Röhreneinheit gelagert ist und die untereinander mithilfe von elastischen Bindungen (76) durch ihre Äste verbunden sind, wobei die Röhren (38, 62) der ersten und der zweiten Einheit zwischen zwei Y (70) gelagert sind.

3. Vorrichtung gemäß Anspruch 1 oder 2, dadurch gekennzeichnet, daß die Röhren (38) der ersten Einheit untereinander so angeschlossen sind, daß die eine Schlange bilden, wobei die Enden der Röhren für Anschlüsse (44) vorgesehen sind, an welchen der Entgaser (36) angeschlossen ist.

4. Vorrichtung gemäß einem der Ansprüche 1 bis 3, dadurch gekennzeichnet, daß die Röhren (62) der zweiten Einheit untereinander so angeschlossen sind, daß sie eine Schlange bilden.

5. Vorrichtung gemäß Anspruch 3, dadurch gekennzeichnet, daß die Anschlüsse (44) an den Röhren der ersten Einheit jeweils mithilfe einer ersten (52a) und einer zweiten (52b) internen Muffe befestigt sind, die entsprechend an den ersten (38a) und den zweiten (38b) Röhren der ersten Einheit montiert sind; erste (54a) und zweite (54b) externe Muffen an jeder von diesen sind entsprechend an den besagten ersten und zweiten Röhren eingebaut, wobei die ersten und zweiten internen Muffen entsprechend an den ersten und zweiten externen Muffen festgemacht sind.

6. Vorrichtung gemäß Anspruch 5, dadurch gekennzeichnet, daß die Röhren (38) der ersten Einheit aus Polyethylen und die internen (52a, 52b) und die externen (54a, 54b) Muffen aus Polypropylen ausgeführt sind.

7. Vorrichtung gemäß einem der Ansprüche 1 bis 6, dadurch gekennzeichnet, daß die Zwischenlagen (70) aus Polypropylen ausgeführt sind.

8. Vorrichtung gemäß einem der Ansprüche 1 bis 7, dadurch gekennzeichnet, daß der Karbonateur ein Karbonateur (12) des zwei Säulen-Typs ist, die eine Eingangssäule (88), in welcher das flüssige Milieu (1) sich mit CO₂ belädt und eine Ausgangssäule (96) umfassen, die mit der Eingangssäule verbunden ist und in welcher das mit CO₂ beladene flüssige Milieu umstürzt.

9. Vorrichtung gemäß Anspruch 8, dadurch gekennzeichnet, daß die Eingangssäule (88) ein das CO₂ in das flüssige Milieu (1) einführende Tauchrohr (92) umfaßt, dessen eintauchendes Ende (94) aus Glas oder gesintertem Inox mit veränderlicher Porosität ist.

10. Vorrichtung gemäß Anspruch 8 oder 9, dadurch gekennzeichnet, daß die Eingangssäule (88) mit einer Aussparung (90) versehen ist, um die Einführung des CO₂ in das flüssige Milieu (1) zu fördern.

11. Vorrichtung gemäß einem der Ansprüche 1 bis 10, dadurch gekennzeichnet, daß der Entgaser (36) eine externe abgeschlossene Kammer (78), die eine Longitudinalachse (79) besitzt, und eine Leitung in U-Form (82) umfaßt, die den Sauerstoff sammelt und dessen einer Arm (84) in der Kammer (78) parallel zur besagten Achse eingebaut ist und dessen Basis (86) an der Seite der Basis (80) der Kammer eingebaut ist, wobei das Ende (85) der den Sauerstoff sammelnden Leitung (82) in den oberen Teil der Kammer mündet; mindestens eine zuführende Leitung (61) und mindestens eine Ausgangsleitung (63) des flüssigen Milieus (1) mündet in den unteren Teil der Kammer.

12. Vorrichtung gemäß einem der Ansprüche 1 bis 11, dadurch gekennzeichnet, daß die Geräte zur Steuerung (66, 67, 68) der zweiten Röhreneinheit (38) pneumatisch sind und dazu dienen, die Röhren (62) der zweiten Einheit entsprechend aufzublasen und zu entleeren, wenn die Temperatur des flüssigen Milieus (1) kleiner als eine erste Einstelltemperatur und größer als eine zweite Einstelltemperatur ist, wobei die erste Einstelltemperatur kleiner ist als die zweite Einstelltemperaur.

13. Vorrichtung gemäß einem der Ansprüche 1 bis 12, dadurch gekennzeichnet, daß der Ausdehnungsbehälter (2) durchsichtig und in Form einer Kammer (100) mit abgerundeten Kanten ausgebildet ist, dessen oberes Ende mit einem Deckel (104) aus Glaserdiamant ist und dessen unteres Ende die Form eines Trichters (102) aufweist.

14. Vorrichtung gemäß Anspruch 13, dadurch gekennzeichnet, daß der Behälter an seinem oberen Teil mit einem Metallgitter (124) aus Glaserdiamant ausgerüstet ist.

15. Vorrichtung gemäß einem der Ansprüche 1 bis 14, dadurch gekennzeichnet, daß sie Geräte (19) zur Regulierung der in den Photobioreaktor (6) eingeführten CO₂-Menge in Abhängigkeit von der Sonnenenergie umfaßt.
